# EUROPÄISCHE PATENTSCHRIFT

(11) **EP 0 574 464 B1**
(45) Veröffentlichungstag und Bekanntmachung des Hinweises auf die Patenterteilung: **29.05.1996**
(21) Anmeldenummer: 92905870.9
(22) Anmeldetag: 04.03.1992
(51) Int. Cl.: A61B 5/04

(54) **VERFAHREN UND VORRICHTUNG ZUM HERAUSFILTERN VON GRUNDLINIENSCHWANKUNGEN AUS EINEM ELEKTROKARDIOGRAMM**
METHOD AND DEVICE FOR FILTERING OUT BASELINE FLUCTUATIONS FROM AN ELECTROCARDIOGRAM
PROCEDE ET DISPOSITIF POUR L'EXTRACTION PAR FILTRAGE D'OSCILLATIONS DE LA LIGNE DE BASE DANS UN ELECTROCARDIOGRAMME

(30) Priorität: 04.03.1991 DE 4106856
(43) Veröffentlichungstag der Anmeldung: 22.12.1993
(73) Patentinhaber: Siemens Elema AB, S-171 95 Solna 1 (SE); SIEMENS AKTIENGESELLSCHAFT, D-80333 München (DE)
(72) Erfinder: LUNDSTRÖM, Lena, S-117 28 Stockholm (SE); KARLSSON, Peter, S-113 37 Stockholm (SE); OHLSSON, Thomas, S-162 43 Vällingby (SE)
(86) Internationale Anmeldenummer: EP9200473
(87) Internationale Veröffentlichungsnummer: WO9215242

(56) Entgegenhaltungen:
- FR-A- 2 254 791
- US-A- 4 781 201
- US-A- 4 887 609
- PATENT ABSTRACTS OF JAPAN vol. 14, no. 215 (C-0716)8. Mai 1990 & JP-A 2 049 644

## Beschreibung

Die Erfindung betrifft ein Verfahren und eine Vorrichtung zum Herausfiltern von Grundlinienschwankungen aus einem Elektrokardiogramm mit Hilfe eines Hoch- oder Bandpaßfilters.

Elektrokardiogramme, die von Patienten abgenommen werden, sind normalerweise von Störsignalen, wie z.B. der von dem Wechselstromnetz induzierten 50 Hz - Wechselspannung, muskelelektrischen Potentialen und Artefakten im Zusammenhang mit der Abnahme des Elektrokardiogramms von dem Patienten überlagert. Diese Störungen, soweit sie im Vergleich zur Herzschlagfrequenz niederfrequenter Art sind, äußern sich in Form von Grundlinienschwankungen bei dem jeweils erfaßten Elektrokardiogramm.

Es ist z.B. aus "Journal of Clinical Engineering", Band 7, Nr. 3, Juli - September 1982, Seiten 235-240 bekannt, diese Grundlinienschwankungen mittels eines Hochpassfilters aus dem erfaßten Elektrokardiogramm herauszufiltern. Dabei ist die untere Grenzfrequenz des Hochpassfilters auf einen Wert festgelegt, der unterhalb der für die Herzsignale charakteristischen Frequenzen bei der niedrigsten vorkommenden Herzfrequenz liegt. Bei hohen Herzschlagfrequenzen ergibt sich daher ein Störungsfrequenzbereich, der nicht von der Hochpassfilterung erfaßt wird.

Der Erfindung liegt die Aufgabe zugrunde, die Effektivität der Grundlinienfilterung zu erhöhen.

Gemäß der Erfindung wird die Aufgabe bezüglich des eingangs genannten Verfahrens dadurch gelöst, daß aus dem Elektrokardiogramm die jeweils aktuelle Herzschlagfrequenz ermittelt wird und in Abhängigkeit davon die untere Grenzfrequenz des Hoch- oder Bandpaßfilters in der Weise verändert wird, daß die Grenzfrequenz bei zunehmender Herzschlagfrequenz erhöht und bei abnehmender Herzschlagfrequenz erniedrigt wird.

Bei der eingangs genannten Vorrichtung ist zur Lösung der Aufgabe vorgesehen, daß die untere Grenzfrequenz des Hoch- oder der Bandpassfilters veränderbar ist, daß der Eingang des Filters mit dem Eingang einer Einrichtung zur Ermittlung der Herzschlagfrequenz aus dem Elektrokardiogramm verbunden ist, und daß diese Einrichtung ausgangsseitig mit einem Steuereingang des Filters zur Steuerung seiner unteren Grenzfrequenz verbunden ist.

Der Vorteil des erfindungsgemäßen Verfahrens bzw. der erfindungsgemäßen Vorrichtung besteht darin, daß durch die Anpassung der unteren Grenzfrequenz des Filters an die jeweils ermittelte Herzschlagfrequenz kontinuierlich der jeweils maximal mögliche Anteil der Grundlinienschwankungen aus dem Elektrokardiogramm herausgefiltert wird.

Die Ermittlung der Herzschlagfrequenz erfolgt in vorteilhafter Weise dadurch, daß das Elektrokardiogramm einer Frequenzanalyse unterzogen wird, oder daß alternativ hierzu das Auftreten von QRS-Komplexen in dem Elektrokardiogramm detektiert wird und aus dem zeitlichen Abstand jeweils aufeinanderfolgender detektierter QRS-Komplexe der Kehrwert gebildet wird.

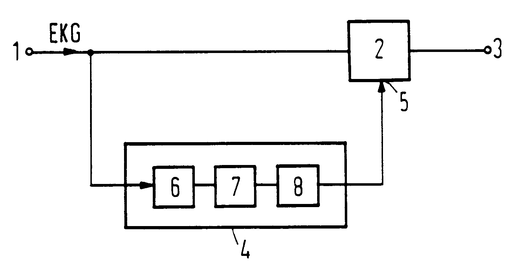

Zur Erläuterung der Erfindung wird im folgenden auf die Figur der Zeichnung Bezug genommen, die ein Blockschaltbild eines Ausführungsbeispiels der erfindungsgemäßen Vorrichtung zeigt.

In der Figur ist mit 1 ein Anschluß bezeichnet, an dem ein von einem hier nicht gezeigten Patienten abgenommenes Elektrokardiogramm (EKG) anliegt. Der Anschluß 1 ist mit dem Eingang eines Hoch- oder Bandpassfilters 2 verbunden, an dessen Ausgang 3 das gefilterte und damit von Grundlinienschwankungen befreite Elektrokardiogramm abgenommen werden kann. Der Anschluß 1 ist ferner mit dem Eingang einer Einrichtung 4 zur Ermittlung der Herzschlagfrequenz aus dem Elektrokardiogramm verbunden; der Ausgang dieser Einrichtung 4 ist mit einem Steuereingang 5 des Filters 2 zur Steuerung der unteren Grenzfrequenz des Filters 2 verbunden. Bei dem gezeigten Ausführungsbeispiel enthält die Einrichtung 4 einen QRS-Detektor 6, mit dem das Auftreten von QRS-Komplexen in dem Elektrokardiogramm (EKG) detektiert wird, eine nachgeordnete Stufe 7 zur Messung des zeitlichen Abstandes zwischen aufeinanderfolgenden detektierten QRS-Komplexen und einen daran anschließenden Kehrwertbildner 8 zur Bildung des Kehrwertes aus dem ermittelten zeitlichen Abstand. Alternativ zu dem gezeigten Ausführungsbeispiel kann die Einrichtung 4 auch aus einem Frequenzanalysator bestehen.

Als Filter 2 kommen insbesondere digitale Filter mit einstellbarer unterer Grenzfrequenz oder eine Reihe von mehreren Filtern mit unterschiedlichen festen Grenzfrequenzen in Betracht, von denen jeweils ein Filter in Abhängigkeit von dem Ausgangssignal der Einrichtung 4 ausgewählt wird. Hoch- bzw. Bandpaßfilter, Frequenzanalysatoren und QRS-Detektoren sind für sich genommen bekannt, so daß eine nähere Beschreibung nicht erforderlich ist.

## Patentansprüche

1. Verfahren zum Herausfiltern von Grundlinienschwankungen aus einem Elektrokardiogramm (EKG) mit Hilfe eines Hoch- oder Bandpassfilters (2), **dadurch gekennzeichnet**, daß aus dem Elektrokardiogramm (EKG) die jeweils aktuelle Herzschlagfrequenz ermittelt wird und in Abhängigkeit davon die untere Grenzfrequenz des Filters (2) in der Weise verändert wird, daß die untere Grenzfrequenz bei zunehmender Herzschlagfrequenz erhöht und bei abnehmender Herzschlagfrequenz erniedrigt wird.

2. Verfahren nach Anspruch 1, **dadurch gekennzeichnet**, daß zur Ermittlung der Herzschlagfrequenz das Elektrokardiogramm (EKG) einer Frequenzanalyse unterzogen wird.

3. Verfahren nach Anspruch 1, **dadurch gekennzeichnet**, daß zur Ermittlung der Herzschlagfrequenz das Auftreten von QRS-Komplexen in dem Elektrokardiogramm (EKG) detektiert wird und aus dem zeitlichen Abstand jeweils aufeinander folgender detektierter QRS-Komplexe der Kehrwert gebildet wird.

4. Vorrichtung zum Herausfiltern von Grundlinienschwankungen aus einem Elektrokardiogramm (EKG) mit einem Hoch- oder Bandpassfilter (2), dessen Eingang mit dem Elektrokardiogramm (EKG) beaufschlagt ist, **dadurch gekennzeichnet**, daß die untere Grenzfrequenz des Filters (2) veränderbar ist, daß der Eingang des Filters (2) mit dem Eingang einer Einrichtung (4) zur Ermittlung der Herzschlagfrequenz aus dem Elektrokardiogramm verbunden ist, und daß diese Einrichtung (4) ausgangsseitig mit einem Steuereingang (5) des Filters (2) zur Steuerung seiner unteren Grenzfrequenz verbunden ist.

5. Vorrichtung nach Anspruch 4, **dadurch gekennzeichnet**, daß die Einrichtung (4) zur Ermittlung der Herzschlagfrequenz ein Frequenzanalysator ist.

6. Vorrichtung nach Anspruch 4, **dadurch gekennzeichnet**, daß die Einrichtung (4) zur Ermittlung der Herzschlagfrequenz einen QRS-Detektor (6), eine nachgeordnete Stufe (7) zur Messung des zeitlichen Abstandes aufeinanderfolgender detektierter QRS-Komplexe und einen daran anschließenden Kehrwertbildner (8) zur Bildung des Kehrwertes aus dem ermittelten zeitlichen Abstand aufweist.

## Claims

1. Method for filtering out baseline fluctuations from an electrocardiogram (ECG) with the aid of a high-pass or band-pass filter (2), characterized in that the respectively current heart rate is determined from the electrocardiogram (ECG) and, as a function thereof, the lower cut-off frequency of the filter (2) is altered in such a way that the lower cut-off frequency is raised as the heart rate increases and is lowered as the heart rate decreases.

2. Method according to Claim 1, characterized in that to determine the heart rate the electrocardiogram (ECG) is subjected to a frequency analysis.

3. Method according to Claim 1, characterized in that to determine the heart rate the occurrence of QRS complexes in the electrocardiogram (ECG) is detected and the reciprocal is formed from the temporal spacing of respectively successive detected QRS complexes.

4. Device for filtering out baseline fluctuations from an electrocardiogram (ECG) having a high-pass or band-pass filter (2), to the input of which the electrocardiogram (ECG) is applied, characterized in that the lower cut-off frequency of the filter (2) is variable, in that the input of the filter (2) is connected to the input of a device (4) for determining the heart rate from the electrocardiogram, and in that this device (4) is connected, on the output side, to a control input (5) of the filter (2) for controlling its lower cut-off frequency.

5. Device according to Claim 4, characterized in that the device (4) for determining the heart rate is a frequency analyser.

6. Device according to Claim 4, characterized in that the device (4) for determining the heart rate has a QRS detector (6), a downstream stage (7) for measuring the temporal spacing of successive detected QRS complexes and a reciprocal former (8), following this stage, for forming the reciprocal from the determined temporal spacing.

## Revendications

1. Procédé pour filtrer des fluctuations de la ligne de base d'un électrocardiogramme (EKG) à l'aide d'un filtre (2) passe-haut ou passe-bande, caractérisé par le fait que la fréquence cardiaque présente est déterminée à partir de l'électro-cardiogramme (EKG) et qu'en fonction de cette fréquence, la fréquence limite inférieure du filtre (2) est modifiée de sorte qu'elle s'élève lorsque la fréquence cardiaque augmente et s'abaisse lorsque la fréquence cardiaque diminue.

2. Procédé suivant la revendication 1, caractérisé par le fait que pour la détermination de la fréquence cardiaque, on soumet l'électrocardiogramme (EKG) à une analyse en fréquence.

3. Procédé suivant la revendication 1, caractérisé par le fait que pour la détermination de la fréquence cardiaque, on détecte l'apparition de complexes QRS dans l'électrocardiogramme (EKG) et qu'à partir de l'intervalle de temps de complexes QRS détectés les uns à la suite des autres, on forme la valeur inverse.

4. Dispositif pour filtrer des fluctuations de la ligne de base d'un électrocardiogramme (EKG) à l'aide d'un filtre (2) passe-haut ou passe-bande à l'entrée duquel on fournit l'électrocardiogramme (EKG), caractérisé par le fait qu'on peut modifier la fréquence limite inférieure du filtre (2), et que l'entrée du filtre (2) est reliée à l'entrée d'un dispositif (4) pour déterminer la fréquence cardiaque à partir de l'électrocardiogramme, et que ce dispositif (4) est relié, en sortie, à une entrée (5) de commande du filtre (2) pour la commande de sa fréquence limite inférieure.

5. Dispositif suivant la revendication 4, caractérisé par le fait que le dispositif (4) pour déterminer la fréquence cardiaque est un analyseur de fréquence.

6. Dispositif suivant la revendication 4, caractérisé par le fait que le dispositif (4) pour déterminer la fréquence cardiaque comporte un détecteur de QRS (6), un étage (7) disposé à la suite pour mesurer l'intervalle de temps entre des complexes QRS détectés successifs et un dispositif (8) de formation de la valeur inverse, relié à cet étage, pour former la valeur inverse à partir de l'intervalle de temps déterminé.
